# EUROPEAN PATENT APPLICATION

(11) **EP 2 275 084 A1**
(43) Date of publication of application: **19.01.2011**
(21) Application number: 09165498.8
(22) Date of filing: 15.07.2009
(51) Int. Cl.: A61K 8/97, A61Q 11/00, A61K 8/04

(54) **Oral care composition**

(71) Applicant: Unilever PLC, 100 Victoria Embankment London EC4Y 0DY (GB)
(72) Inventor: Ashcroft, Alexander Thomas, Bebington, Wirral, Merseyside CH63 3JW (GB)
(74) Representative: James, Helen Sarah

(57) **Abstract**

An oral care composition comprising from 0.1 to 35% by weight of bamboo granules having a median particle size of less than 2•10⁻⁴ m and a carrier for said bamboo granules.

## Description

This invention relates to an oral care composition comprising a superior abrasive system, in particular an abrasive system comprising small particle size bamboo granules.

The inclusion of abrasives in oral compositions such as toothpaste is well known. The abrasive has a cleaning as well as a polishing/whitening benefit. The removal of tartar from the tooth surface is thought to reduce the incidence of caries as well as enhancing the visual appearance of the teeth.

Dental practitioners recommend that tartar removal should be exercised professionally at least twice a year, preferably more, but it is recognised that the build-up of tartar can be reduced by effective brushing of the teeth.

Typical abrasives used in toothpastes include silicas and chalk, however, the prior art also discloses the use of other abrasives in toothpaste formulations.

WO 07/078631 (Huber) discloses dentifrices comprising biogenic silica materials that exhibit excellent abrasive qualities. The biogenic silica is preferably derived from rice husks.

The abrasivity of a toothpaste is measured according to a protocol described in the journal of Dental Research (1976) 55(4), 563. This describes how the Relative Dental Abrasion (RDA) and Relative Enamel Abrasion (REA) are evaluated. RDA values referred to in this specification are determined using the protocol described in the journal of Dental Research (1976) 55(4), 563.

Ideally, a toothpaste is capable of cleaning the teeth without wearing down the tooth enamel and dentine too much. So, while it is necessary to have a cleaning efficacy, a toothpaste with too high an RDA is undesirable.

The cleaning property of oral care compositions is typically expressed in terms of Pellicle Cleaning Ratio (PCR) value. The PCR test measures the ability of a composition to remove stained pellicle film from a tooth under fixed brushing conditions. PCR values referred to in this specification are determined using the test described by M. J. Pickles et al, in Int. Dent. J., 197-202 (2005).

According to a first aspect of the invention, there is provided an oral care composition comprising from 0.1 to 35% by weight of bamboo granules having a median particle size of less than 200 microns and a carrier for said bamboo granules.

According to a second aspect of the invention, there is provided a method of cleaning teeth comprising the use of an oral care composition according to the first aspect of the invention.

According to a third aspect of the invention, there is provided the use of bamboo granules having a median particle size of less than 200 microns for the manufacture of a dentifrice for cleaning teeth. The use of toothpastes in accordance with the invention may lead to improved shine or gloss for the treated teeth.

According to further aspects of the invention, there is provided the use of bamboo granules having a median particle size of less than 200 microns for the manufacture of a dentifrice for alleviating oral health problems such as dental erosion and caries.

The bamboo granules used in the present invention have a median particle size (d50) of less than 200 microns, preferably less than 120 microns, more preferably 70 microns or less, and most preferably 15 microns or less. Such small particle size bamboo granules are found to give superior cleaning. To retain a good cleaning performance and yet avoid the need for excessive milling of the bamboo granules, a median particle size of from 5 to 100, preferably 5 to 75, and more preferably 10 to 50 microns may be used.

Particle size definitions, including the median particle size, should all be understood to be "by volume". Thus, at the "median particle size", 50% by volume of the sample has a higher size and 50% by volume of the sample has a lower size. Such measurements may be made laser diffraction, using an instrument such as a Sympatec OASIS particle size analyser, supplied by Sympatec GmbH.

It is preferred that the bamboo granules have a certain percentage of granules having a particle size of less than 20 microns. Preferably, this percentage is at least 25% and more preferably it is at least 50%. These percentages should be considered to be by volume, as mentioned in the above paragraph. In particularly preferred embodiments, the percentage of granules less than 20 microns may be greater than 75%, or even greater than 95%, by volume.

The bamboo granules are typically prepared by milling, i.e. grinding down, a bamboo stem or part thereof, preferably from the medulla of bamboo stems. The medulla of bamboo stems is especially effective because it has a high content of silica in its cell walls. Spiny bamboo medulla is a particular source of bamboo granules suitable for use in accordance with the invention.

The carrier for the bamboo granules may be any material suitable for use in the mouth. Typically, the carrier will be aqueous based. A preferred carrier is an aqueous solution of a humectant. Humectants serve to add body or "mouth texture" to an oral care composition as well as preventing it from drying out. Suitable humectants include polyethylene glycol, propylene glycol, glycerol, erythritol, xylitol, sorbitol, mannitol, lactitol, and hydrogenated starch hydrolysates. Sorbitol is a preferred humectant for use in compositions according to the invention.

Compositions according to the invention typically have a total amount of humectant of from 10 to 90%, preferably from 30 to 70%, and more preferably from 40 to 60% by weight.

Compositions according to the invention may comprise another particulate abrasive in addition to the bamboo granules. Materials such as silicas, aluminas, calcium carbonates, dicalciumphosphates, calcium pyrophosphates, hydroxyapatites, trimetaphosphates, insoluble hexametaphosphates, including agglomerated particulate abrasive materials, may be used.

Compositions according to the invention typically have a total amount of abrasive, whether bamboo extract or other material as described above, of from 1 to 50%, preferably from 2 to 35%, and more preferably from 5 to 25% by weight.

The bamboo granules according to the invention comprise from 0.1 to 35%, preferably from 1 to 20%, and more preferably from 5 to 15% by weight of the composition.

Compositions according to the invention have been found to give good cleaning performance, particularly in terms of PCR, by use of an abrasive that is natural and from renewable sources. Further, compositions according to the invention may have RDA values that are reasonably low. Preferably, compositions according to the invention give PCR values of 40 or greater, more preferably 45 or greater, and most preferably 50 or greater. It is further preferred that the RDA value of the composition is numerically lower than its PCR value.

A preferred additional component in the compositions of the invention is a surfactant. Surfactants make the composition more cosmetically acceptable. The surfactant is preferably a detersive material which imparts to the composition detersive and foaming properties. Suitable surfactants include anionic, cationic, non-ionic, zwitterionic, amphoteric, and betaine surfactants. Anionic surfactants such sodium lauryl sulphate are particularly suitable. The total amount of surfactant in the composition is typically from 0.1 to 15%, preferably from 0.2 to 5%, and more preferably from 0.3 to 3%, by weight.

A thickening agent is a useful a component in compositions according to the invention. Such agents provide a gelatinous structure that stabilises the composition against phase separation. Suitable thickening agents include silica thickener; starch; gums such as gum Arabic, xanthan gum, guar gum and cellulose gum; magnesium aluminium silicate; carrageenan; sodium alginate; agar-agar; pectin; gelatin; cellulose compounds such as cellulose, carboxymethyl cellulose, and hydroxyethyl cellulose; natural and synthetic clays such as hectorite clays. Amorphous precipitated silica makes a particularly suitable thickening agent. The total amount of such agents is typically from 1 to 20%, more typically 5 to 15%, by weight of the composition.

The composition according to the invention may be any oral care composition or dentifrice, but is typically a toothpaste, optionally in the form of a gel or an opaque paste.

The composition according to the invention may also comprise other ingredients which are common in dentifrices. Examples of such ingredients include:
antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium-, calcium-, magnesium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamin C;
plant extracts;
desensitising agents, e.g. potassium citrate, potassium chloride, potassium tartrate, potassium bicarbonate, potassium oxalate, potassium nitrate and strontium salts;
anti-calculus agents, e.g. hypophosphite-containing polymers, organic phosphonates and phosphocitrates etc.;
gum protection agents, e.g. vegetable oils such as sunflower oil, rape seed oil, soybean oil and safflower oil; silicone oil; and hydrocarbon oil. The gum protection agent may be an agent capable of improving the permeability barrier of the gums;
biomolecules, e.g. bacteriocins, antibodies, enzymes, etc.;
flavours, e.g. peppermint and spearmint oils;
preservatives, e.g. phenoxyethanol or benzyl alcohol;
opacifying agents, e.g. titanium dioxide;
colouring agents;
pH-adjusting agents, e.g. sodium hydroxide or sodium bicarbonate;
sweetening agents;
buffers, e.g. trisodium phosphate, pentasodium triphosphate, or glycine; and
other optional ingredients that may be included are e.g. bleaching agents such as peroxy compounds e.g. potassium peroxydiphosphate, effervescing systems such as sodium bicarbonate/citric acid systems, colour change systems, and so on.

### Examples

The formulation detailed in Table 1 may be prepared by methods known in the art.

**Table 1**

| **Component** | **% w/w** |
|---|---|
| Polyethylene glycol (PEG) 32 | 4.07 |
| Bamboo granules | 8.00 |
| Flavour | 1.12 |
| Thickening silica | 8.38 |
| Sorbitol (70% ad.) | 64.15 |
| Sodium lauryl sulphate | 1.83 |
| Sodium saccharin | 0.25 |
| Sodium carboxymethylcellulose | 0.61 |
| Sodium fluoride | 0.33 |
| Colour | 0.002 |
| Titanium dioxide | 0.05 |
| Water | To 100 |

Formulations in accordance with Table 1, diluted to a slurry with water (*vide infra*), were prepared and tested. A range of bamboo granule samples, each having a different particle size distribution as detailed in the first and second columns of Table 2, was used. Each of the bamboo granule samples used in slurries 1 to 4 was prepared from the bamboo granule sample used in slurry A, by milling in a simple coffee grinder. The third column of Table 2 indicates the duration of the milling for each bamboo granule sample. The bamboo granule sample used for slurry A was a commercial sample of spiny bamboo medulla granules, obtained as a white powder by grinding the medulla of bamboo stems.

**Table 2**

| **Bamboo granules particle size and milling details** | | | **Slurry number** | **PCR 800** |
|---|---|---|---|---|
| **d50 (µ)** | **amount less than 20µ by volume** | **Milled for** | | |
| 289 | 0.5% | 0 mins. | A | 24.4 |
| 119 | 34% | 2 mins. | 1 | 50.4 |
| 70 | 25% | 3 mins. | 2 | 44.8 |
| 15 | 55% | 4 mins. | 3 | 60.6 |
| 10 | 62% | 5 mins. | 4 | 57.7 |

The slurries for testing were prepared by mixing 30.5g of the toothpaste according to Table 1 (prepared minus the bamboo granules, by conventional means) with 8g of the bamboo granule sample and 61.5g of distilled water. The slurries were stirred using a Heidolph end over mixer until homogeneous. The PCR 800 values of the slurries were measured using the method of M. J. Pickles et al described in Int. Dent. J., 197-202 (2005). The "800" refers to the number of strokes employed.

It can be seen from the PCR values given in Table 2, that compositions comprising the smaller particle size bamboo samples gave superior cleaning.

The compositions according to the invention detailed in Table 3 may be prepared by methods known in the art.

**Table 3**

| **Component** | **% w/w** | | | | |
|---|---|---|---|---|---|
| Example: | **5** | **6** | **7** | **8** | **9** |
| PEG 32 | 4 | 1.3 | 5 | 2 | 4 |
| Bamboo granules | 4 | 40 | 10 | 10 | 6 |
| Abrasive silica | 4 | -- | 2 | -- | -- |
| Perl ite | -- | -- | 3 | -- | -- |
| Flavour | 1.1 | 1.2 | 1.2 | 1 | 1.1 |
| Thickening silica | 8.4 | 5.5 | 8 | 9 | 8.5 |
| Sorbitol (70% ad.) | 64 | 41 | 40 | 25 | 63 |
| Cocamidopropyl betaine (CAPB) | -- | -- | -- | -- | 1 |
| Sodium lauryl sulphate | 1.8 | 1.8 | 1.8 | 1.8 | 1 |
| Sodium saccharin | 0.25 | 0.28 | 0.25 | 0.2 | 0.25 |
| SCMC | 0.61 | 0.4 | 0.8 | 1 | 0.9 |
| Sodium fluoride | 0.33 | -- | 0.32 | 0.31 | 0.3 |
| SMFP | -- | 1.11 | -- | -- | -- |
| Colour | 0.002 | -- | -- | -- | -- |
| Titanium dioxide | 0.05 | 0.05 | 1 | -- | 0.05 |
| Sodium hydroxide | -- | -- | -- | -- | 0.06 |
| Sodium bicarbonate | -- | -- | -- | -- | 2 |
| Glycine | -- | -- | -- | -- | 0.35 |
| Preservative | -- | 0.3 | 0.3 | 0.4 | -- |
| Pentasodium triphosphate | -- | -- | 0.5 | -- | -- |
| Trisodium phosphate | -- | 1.1 | -- | -- | -- |
| Water | To 100 | To 100 | To 100 | To 100 | To 100 |

## Claims

1. An oral care composition comprising from 0.1 to 35% by weight of bamboo granules having a median particle size of less than 200 microns and a carrier for said bamboo granules.

2. An oral care composition according to claim 1, wherein the carrier is an aqueous solution of humectant.

3. An oral care composition according to claim 1 or 2, comprising a thickener agent.

4. An oral care composition according to any preceding claim, comprising an anionic surfactant.

5. An oral care composition according to any preceding claim, comprising an additional abrasive agent.

6. An oral care composition according to any preceding claim, wherein the bamboo granules are derived from the medulla of bamboo stems.

7. An oral care composition according to claim 6, wherein the bamboo granules are derived from spiny bamboo medulla.

8. An oral care composition according to any preceding claim, wherein the bamboo granules have a median particle size of from 5 to 100 microns.

9. An oral care composition according to any preceding claim, wherein the bamboo granules have a percentage of granules having a particle size of less than 20 microns that is at least 25%.

10. An oral care composition according to any preceding claim, having a median particle size of less than 15 microns.
